Europäisches Patentamt

European Patent Office

Office européen des brevets

(11) Publication number: **0 474 891 A1**

## EUROPEAN PATENT APPLICATION

(21) Application number: 90117344.3

(22) Date of filing: 08.09.90

(51) Int. Cl.⁵: **C12N 15/62**, C12N 15/30, C12N 15/44, C12N 15/31, C12N 1/21, //A61K39/112, A61K39/015,A61K39/145

(43) Date of publication of application:
**18.03.92 Bulletin 92/12**

(84) Designated Contracting States:
**AT BE CH DE DK ES FR GB GR IT LI LU NL SE**

(71) Applicant: **BEHRINGWERKE
Aktiengesellschaft
Postfach 1140
W-3550 Marburg 1(DE)**

(72) Inventor: **Schorr, Joachim
Bauerbacher Str. 51
3550 Marburg-Bauerbach(DE)**
Inventor: **Knapp, Bernhard, Dr.
Baumgarten 9
3550 Marburg 13(DE)**
Inventor: **Hundt, Erika, Dr.
Zum Hirtzborn 8
3550 Marburg-Wehrshausen(DE)**
Inventor: **Küpper, Hans, Dr.
Wannkopfstr. 12
3550 Marburg(DE)**
Inventor: **Amann, Egon, Dr.
Sachsenring 6
3550 Marburg-Wehrda(DE)**

(74) Representative: **Klein, Otto, Dr. et al
Hoechst AG Zentrale Patentabteilung
Postfach 80 03 20
W-6230 Frankturt am Main 80(DE)**

(54) **Vectors for expression of malarial antigens on the surface of Salmonella vaccine strains.**

㊼ Recombinant expression vectors are described which can be used for the expression of the malarial antigens HRPII or SERP or of parts thereof on the surface of gram-negative bacteria being capable of inducing a humoral immune response in mammals. Furthermore, corresponding Salmonella-based vaccines are described.

The invention relates to new recombinant expression vectors which are capable of expressing immunogenic peptides of the malarial antigens HRPII or SERP on the surface of Salmonella vaccine strains. The invention furthermore relates to a vaccine containing said Salmonella strains and inducing a humoral immune response in mammals by oral vaccination.

The malarial antigens HRPII and SERP are characterized in EP-A2 0 315 085, Wellems and Howard, Proc. Natl. Acad. Sci. USA 83 (1986), 6065 - 6069, Knapp et al., Behring Res. Commun. 82 (1988), 349 - 359, EP-A1 0 283 882, Knapp et al., Mol. Biochem. Paras. 32 (1989), 73 - 84, and Higgins et al., Nature 340 (1989), 604. Although some of these documents also disclose vaccines containing said antigens or immunologically active parts thereof, there is still a need to provide improved vaccines suitable for eliciting a protective and reliable humoral immune response against malaria-causing protozoa, such as Plasmodium falciparum (P. falciparum).

Transformed bacterial strains which are applicable as life vaccines are disclosed in DE-A 38 28 666 and EP-A 0 357 208. Hackett, Vaccine 8 (1990), 5-11, summarizes the state of the art in the field of Salmonella-based vaccines.

The technical problem underlying the present invention is to provide genetically engineered Salmonella strains which are suitable for a protective and reliable prophylaxis of malaria by inducing a high level humoral immune response after oral application.

The solution of the above technical problem is achieved by providing the embodiments characterized in the claims.

Accordingly the present invention provides a recombinant expression vector comprising the following components:

(a) a strong inducible promoter;

(b) a hybrid gene encoding a hybrid OmpA protein of gram-negative bacteria;

(c) a cloning site located in the part of the OmpA gene corresponding to the third external loop of the OmpA protein; and

(d) a DNA sequence which is

(da) inserted in frame into the cloning site (c), and

(db) encodes the malarial antigen HRPII or SERP or a part thereof being capable of inducing a humoral immune response in a mammal, said malarial antigen being preferably derived from Plasmodium falciparum.

The term "strong inducible promoter" refers to a nucleotide sequence which on the transcriptional level controls the expression of a protein encoded by a sequence localized downstream with high efficiency, and which (a process which) can be induced either by a temperature-shift or by addition of a chemical substance (e.g. IPTG). A preferred example of such a strong inducible promoter is the tac-promoter which is under the control of the lac-repressor.

The term "cloning site" refers to a DNA sequence containing a restriction enzyme cleavage site which is suitable for the incorporation of a desired DNA fragment.

The term "a part of the malarial antigen HRPII or SERP being capable of inducing a humoral immune response in a mammal" refers to regions of HRPII or SERP which are useful for the production of vaccines. Such regions can be determined by a person skilled in the art according to conventional methods, e.g. by applying conventional computer programs (T-cell epitopes can be confirmed experimentally). In the case of SERP such regions are preferably regions being homologous to SH proteases or which contain T-cell epitopes. In this context the term "SH protease" refers to a protease carrying an essential cysteine residue in its active site. Furthermore, the term "T-cell epitope" refers to an amino acid sequence stimulating a cellular immune response mediated by T-lymphocytes.

The fusion proteins encoded by the recombinant expression vector of the present invention are transported upon expression to the bacterial cell wall and are inserted in such a way as to present the malarial antigens HRPII or SERP or said part thereof to the outer environment. This presentation is effected in such a way that the immune system of a mammal exposed to such transformed bacterial hosts recognizes said malarial antigen and raises a protective immune response. Preferably said malarial antigens are presented on the surface of the transformed bacterial hosts of the present invention in a conformation similar to their native form.

In a preferred embodiment of the present invention, the recombinant expression vector additionally comprises:

(ca) inserted into said cloning site (c) a DNA sequence encoding amino acids 1 to 48 and 279 to 515 of the Influenza hemagglutinin (HA) protein, containing a first cloning site at a position encoding amino acid 46 of the HA protein and the second cloning site at a position encoding amino acid 400 of the HA protein, wherein said DNA sequence (d) is inserted into at least one of said cloning sites of the DNA sequence (ca).

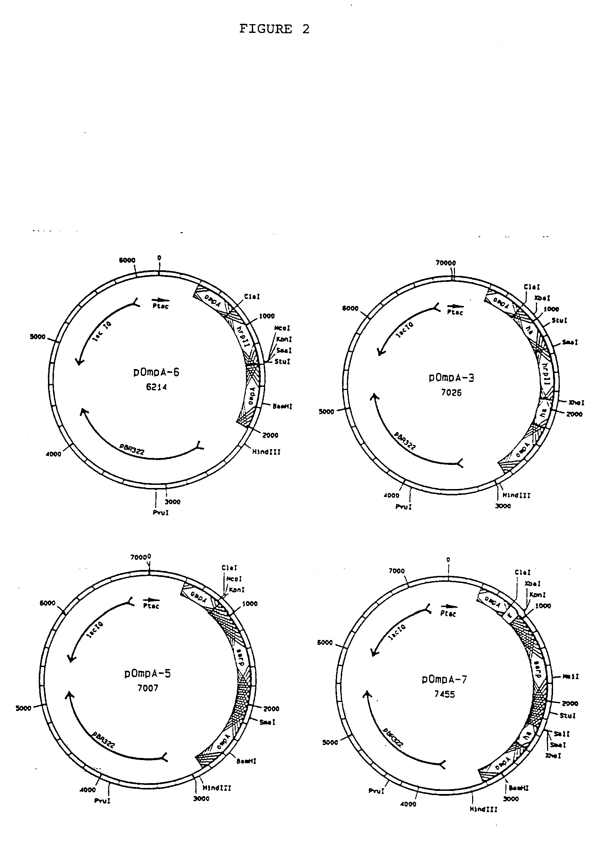

In the further preferred embodiment of the present invention said part of HRPII consists of the 189 amino acids contained in the recombinant expression vector pOmpA-6 having the restriction map given in Fig. 2.

In another preferred embodiment of the present invention the recombinant expression vector encodes a part of HRPII which consists of the 177 amino acids contained in the recombinant expression vector pOmpA-3 having the restriction map given in Fig. 2.

In another preferred embodiment of the recombinant expression vector of the present invention said part of SERP contains a region being homologous to SH proteases.

In a particularly preferred embodiment the region being homologous to SH proteases contains amino acids 442 to 892, 573 to 595, or 745 to 787 of SERP.

In a further preferred embodiment of the recombinant expression vector of the present invention said part of SERP contains at least two T-cell epitopes.

In a particularly preferred embodiment this part containing T-cell epitopes contains amino acids 442 to 892 or 640 to 700 of SERP.

Further preferred embodiments of the recombinant expression vector of the present invention are characterized in the claims.

The present invention also relates to Salmonella vaccine strains which are transformed with any of the recombinant expression vectors of the present invention.

In a preferred embodiment said Salmonella vaccine strains are derived from Salmonella typhi or Salmonella typhimurium. In this context it has to be understood that Salmonella typhimurium is most infective for mice and that its vaccination properties in mice are often used in the art as a model system for Salmonella typhi and its vaccination properties in humans.

Nucleotide sequences encoding 189 amino acids of the malarial HRPII antigen and encoding 451 amino acids of the malarial SERP antigen were cloned in frame with the polylinker of expression vectors containing a hybrid OmpA gene generated by recombinant DNA technology from the OmpA genes of the gram-negative bacteria E. coli and Shigella dysenteriae. Transformation of S. typhimurium vaccine strains with these recombinant vectors and induction of expression results in expression of said malarial antigens or the parts thereof on the surface of the transformed bacteria. Oral vaccination of NMRI mice with transformed S. typhimurium vaccine strains of the present invention results in considerable humoral immune response against the respective malarial antigens.

In a further embodiment the present invention relates to a recombinant fusion protein capable of inducing a humoral immune response in mammals which is expressed by any of the Salmonella vaccine strains of the present invention.

In another embodiment the present invention relates to a vaccine for the prophylaxis of malaria containing a Salmonella vaccine strain of the present invention or a recombinant fusion protein of the present invention. Optionally these immunologically active compounds are administered in the vaccine of the present invention in combination with a pharmaceutically acceptable carrier and/or diluent.

In a preferred embodiment the vaccine of the present invention is orally applicable. Thus, the vaccine of the present invention is preferably provided in a form that protects the immunologically active components from gastric acid.

In a further embodiment the present invention relates to a method for the prophylaxis of malaria comprising administering to a mammal in need thereof a dosage of a Salmonella vaccine strain or a recombinant fusion protein of the present invention, said dosage being suitable for inducing a protective humoral immune response.

The figures show:

Fig. 1: (A) Polylinker region of plasmid pHS164-L. The sequence of the original plasmid pHS164 is shown in small letters. An oligonucleotide has been inserted between a ClaI and a StuI restriction site to form pHS164-L.

(B) Linker I and Linker II within the HA coding region of plasmid pinf4-49.

Fig. 2: Structure of OmpA fusion plasmids of the present invention. Coding regions and sizes of OmpA, HA, HRPII and SERP fused genes are shown. Relevant restriction sites, in particular those at gene fusion junctions are indicated.

Fig. 3: Coomassie blue and Western blot analysis of OmpA fusion proteins of the present invention.

Fig. 3A shows a Coomassie blue stained 9 % SDS-polyacrylamid gel with membrane fractions of Salmonella strains harbouring the following plasmids: lane 1, pHS164-L; lane 2, pinf4-49; lane 3, pOmpA-3; lane 4, pOmpA-5; lane 5, pOmpA-6; lane 6, pOmpA-7.

Fig. 3B-D show Western blot analyses of the same samples as in Fig. 3A probed with a anti-OmpA C-terminal peptide serum (B), and anti-SERP serum (C) or an anti-HRPII serum (D), respectively.

Fig. 4: Immunofluorescence of intact cells of S. typhimurium strain SR-11 harbouring pOmpA-3 probed with an anti-HRPII serum.

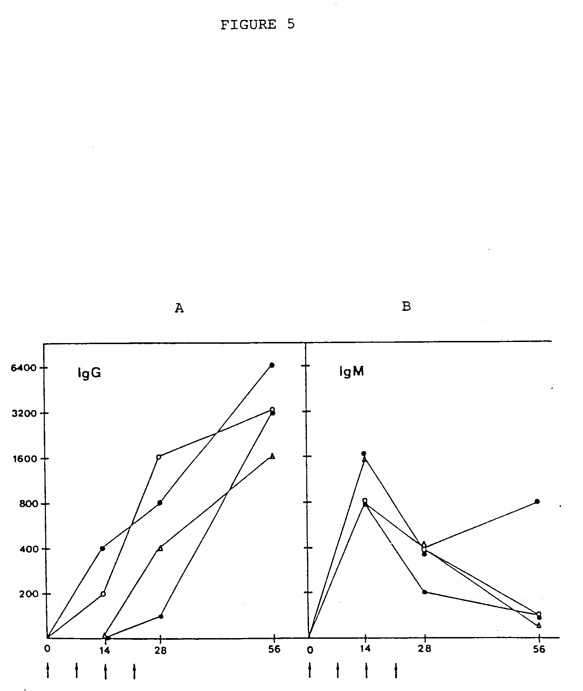

Fig. 5: Antibody response of mice immunized orally with S. typhimurium strain SR-11 expressing HRPII and SERP OmpA fusion proteins at its surface. Mice were orally immunized at days 0, 7, 14, 21 (indicated by arrows). Blood samples were taken at days 0, 14, 28 and 56, pooled, and tested for the presence of HRPII and SERP specific antibodies by ELISA. For Ig typing, anti-IgG (A) and anti-IgM (B) specific POD-conjugated goat antisera were used. Asterisk = pOmpA-3; open circle = pOmpA-6, triangle = pOmpA-5; filled circle = pOmpA-7. The titer refers to the highest dilution of test serum at which the ratio of $A_{492}$ of test serum to $A_{492}$ of preimmune serum was > 2.0.

Fig. 6: Western blot analysis of P. falciparum schizont extracts probed with mouse antisera obtained after oral immunization. Lanes 1, 2, 4, 5: Sera (1:100) taken at day 56 from mice immunized orally with S. typhimurium harbouring pOmpA-3 (1), pOmpA-6 (2), pOmpA-5 (4) and pOmpA-7 (5). Rabbit antisera (1:3000) raised against MS2 fusion proteins of HRPII (3) and SERP (6) partial sequences were used as reference.

Fig. 7 Nucleotide and derived amino acid sequence of the 583 bp HRP II cDNA fragment (capital letters). Sequences of the vector pUC8 are denoted by small letters.
The C nucleotide denoting the 5' end of the Bal 31 digest used for the construction of pOmpA-3 is marked by an asterisk. Restriction sites relevant for construction of vectors pOmpA-3 and pOmpA-6 are shown.
The oligonucleotides used for the PCR reaction in the construction of pOmpA-6 correspond to the underlined sequences.

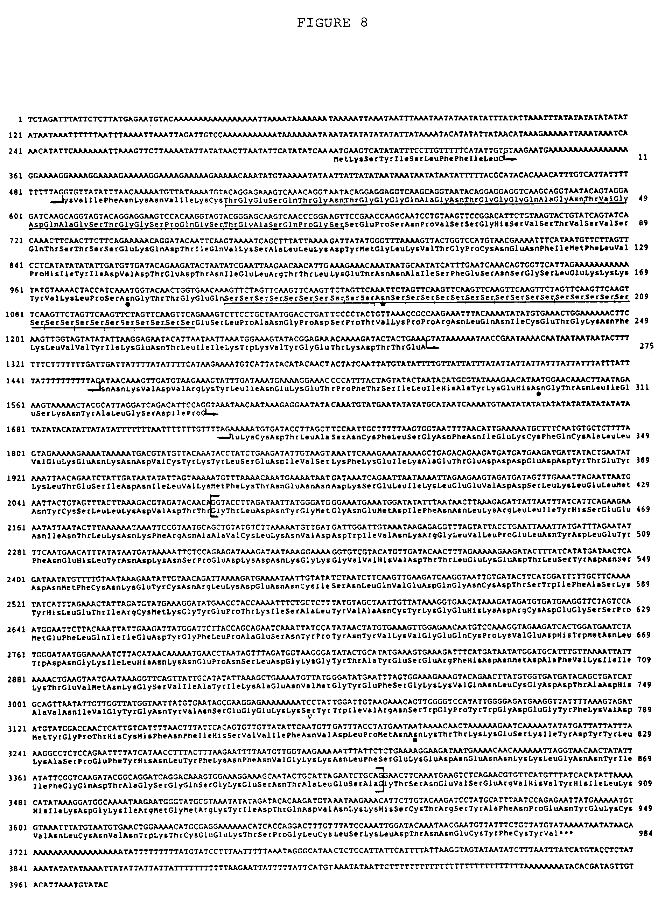

Fig. 8 Nucleotide and derived amino acid sequence of the complete SERP gene.
The 1.3 kb DNA fragment isolated by Kpn I and Pst I digestion (construction of pOmpA-5) or amplified by PCR (the amplified fragment used for the construction of pOmpA-7) is shown in brackets. The dots indicate potential glycosylation sites. Introns are marked by arrows. Underlined regions represent repetitive elements.

The following examples illustrate the invention. Additional information on the applied recombinant DNA techniques can be found in Sambrook et al., "Molecular Cloning", Cold Spring Harbour Laboratory, Cold Spring Harbour, 2nd edition, 1989.

**Example 1**

Construction of OmpA vectors carrying fragments of the genes encoding HRPII and SERP

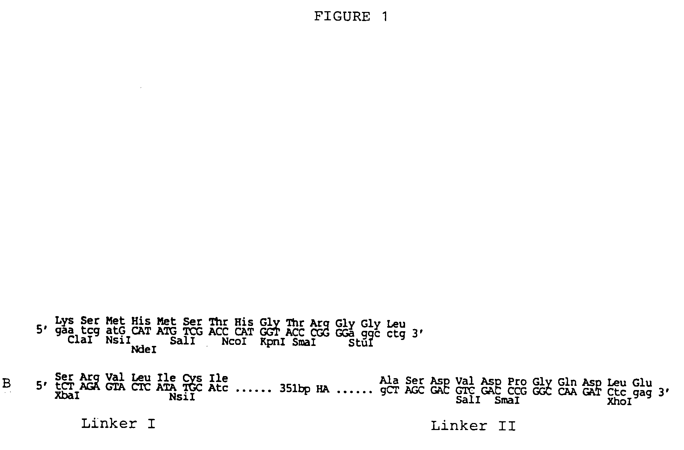

The OmpA expression vectors pHS164 and pinf4-49 have been constructed by S. Pistor and G. Hobom (S. Pistor and G. Hobom, Klin. Wochenschr. 66 (1988), 110-116, DE-A 38 28 666). pHS164 encodes a hybrid OmpA protein consisting of amino acids 1-46 from the E.coli OmpA protein and of amino acids 47-233 from the Shigella dysenteriae OmpA protein. In order to provide for more cloning sites, a polylinker with the sequence 5'-ATCGATGCATATGTCGACCCATGGTACCCGGGGAGGCCT-3' which carries unique restriction sites for ClaI, NsiI, NdeI, SalI, NcoI, KpnI, SmaI and StuI has been inserted between the unique ClaI and StuI sites of pHS164 yielding pHS164-L (Fig. 1a). All the necessary cloning steps were done in E. coli K12 A490 (recA, lac, met, thr, rk-, mk-). Isolation of plasmid DNA, preparation of DNA fragments, ligation and transformation of E. coli cells were carried out using standard methods (Sambrook et al., loc. cit.). Nucleotide sequencing on double-stranded DNA was performed using the protocol of USB (Cleveland, Ohio) based on the dideoxy termination method. The OmpA protein encoded by pHS164-L carries 9 additional amino acids in its third external loop. Insertion of foreign DNA matching the translational reading frame from the OmpA protein into the polylinker thus results in its expression in the third external loop of OmpA. Plasmid pinf4-49 encodes a stem-like structure in the third external loop of OmpA formed by the hydrophobic segments consisting of amino acids 1-48 and 279-514 of the influenza HA protein. Two cloning sites are present: linker I at a position corresponding to codon 46 of the HA gene and linker II at a position corresponding to codon 400 of the HA gene (Fig. 1b, DE-A 38 28 666). Cloning of DNA fragments between both linkers was intended to improve the exposition of the antigen expressed.

In the OmpA vectors transcription of the OmpA gene is under the control of the tac-promoter, whose activity is controlled by the lac-repressor. The lacIQ allele itself is present on the vectors and therefore the lac-repressor will be provided in cis, irrespective of the actual genetic background of the bacterial strains.

4

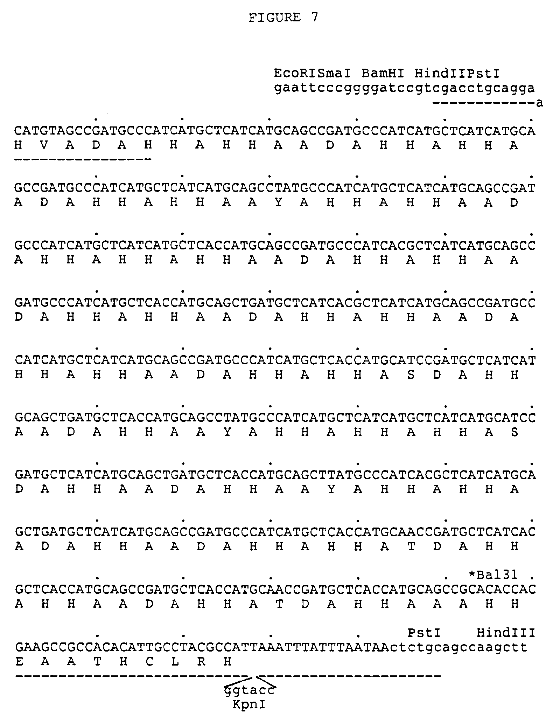

Employing the vectors pHS164-L and pinf4-49, four expression plasmids were constructed (Fig. 2): pOmpA-3: pSK-HRPII which carries a 0.55 kb small SmaI-XhoI-cDNA fragment (Fig. 7) from which the translational stop-codon was deleted, was digested with SmaI and XhoI. This fragment was isolated and cloned into the respective sites of pinf4-49. More specifically, the following steps were carried out:

1. Digestion of pUC8-HRPII DNA with Hind III.
2. Digestion with Bal 31 according to Sambrook et al. (loc. cit.).
3. Fill-in reaction with Klenow enzyme.
4. Fill-in reaction with T4 DNA polymerase.
5. Digestion with EcoRI resulting in the liberation of the insert.
6. Gel electrophoresis of DNA fragments and isolation of DNA fragments which are 30-80 bp shorter than the insert DNA.
7. Digestion of Bluescript pSK (+) vector DNA with Hind II and EcoRI.
8. Ligation of the Bal 31 fragments into Hind II/EcoRI digested pSK (+).
9. Transformation of XL1 blue cells with the recombinant vector.
10. DNA minipreps analyzed with XhoI and EcoRI.
11. Sequencing of miniprep DNAs from 20 different clones: none of the DNA fragments was in frame with the vector pinf4-49 at the XhoI site.

One of the clones had the following sequence at the 3' cloning site of HRPII:

```
        XhoI            HpaII
    _____       _____
    CTC GAG       GTC│CGG        TGC ATG

        pSK(+)           │       HRPII 3' end
```

The XhoI site of this construct is not in frame with the XhoI site of the pinf4-49 vector. By this cloning step a new HpaII restriction site is created.

12. Digestion of the DNA of this clone with KpnI and EcoRI.
13. Isolation of the insert DNA.
14. Restriction digestion of insert DNA with HpaII.
15. Isolation of a 600 bp EcoRI-HpaII fragments by gel electrophoresis.
16. Ligation of the insert into pSK (+) digested with AccI and EcoRI.

The following scheme summarizes the strategy for deleting the 5' C nucleotide of the HpaII site.

a) Digestion of recombinant vector with HpaII

```
        XhoI            HpaII
    _____        ↓
    CTC GAG       GTC│CGGC        TGC ATG

        pSK(+)           │        HRPII
```

b) Digestion of vector pSK (+) with AccI

```
        XhoI            AccI
    _____       ____
    CTC GAG       GTC GAC GGT
                    ↓
        pSK (+)
```

c) Ligation of the 600 bp EcoRI HpaII fragment of HRPII into pSK (+) digested with AccI and EcoRI.

5

```
         XhoI
         ‾‾‾‾‾‾‾‾
         CTC GAG      GTC GGC      TGC ATG
                         │
                         │
         pSK(+)          │            HRPII
                         │
```

Deletion of the base C in the HPRII site results in a new recombinant vector in which the XhoI site of the pSK-HRPII plasmid is now in frame with the XhoI site of the vector pinf4-49.

17. Conformation of the new sequence (deletion of the base C at the 3' end of HRPII) by sequencing.

18. Restriction digestion with SmaI and XhoI and isolation of a 600 bp fragment.

19. Cloning of this 600 bp fragment into pinf4-49 digested with SmaI and XhoI results in new recombinant vector pOmpA-3.

pOmpA-5: pUC18-SERP which carries the complete SERP gene on a 5.8 kb XbaI fragment (Knapp et al., Mol. Biochem. Parasitol. 32 (1989), 73-84) was digested with KpnI and PstI (Fig. 8). A 1.3 kb fragment was isolated and ligated into the respective sites of pSK vectors (Stratagene). Subcloning of the SERP fragment into pSK was performed in order to create the correct translational reading frame at the 3' end of the SERP coding region. The resulting plasmid pSK-SERP was digested with SmaI and KpnI, the 1.3 kb fragment was isolated and cloned into the respective sites of pHS164-L.

pOmpA-6: The oligonucleotide sequences (1: 5'-CGACCTGCAGGAACATGTAGCCGATGCCC-3' and 2: 5'-GCAGAGTTATTAAATAAATTTGGTACCATGGCGTAGGCAATGTGTGGCGGCTTC-3') were used together with the plasmid pUC8-HRPII (Knapp et al., Behring Res. Commun. 82 (1988) 349-359) to amplify a 0.6 kb fragment (Fig. 7) by PCR according to published procedures (Saiki et al., Science 230 (1988), 1350-1354). This fragment was cloned into the NsiI and KpnI sites of the pHS164-L vector after digestion with PstI and KpnI.

pOmpA-7: Two oligodeoxynucleotides (1: 5'-AAGGGAACAAAATCTAGAGGTACCTTAGATAATTATGGG-3' and 2: 5'-CTAGTGGATCCCGTCGACTGCAGATTCTAATGCAGTATTGCT-3') were used together with pSK-SERP for PCR. The resulting 1.3 kb ( Fig. 8) fragment was digested with XbaI and SalI and cloned in frame between these sites into pinf4-49.

The resulting plasmids pOmpA-3 and pOmpA-6 encode thus 177 or 189 amino acids, respectively, of the HRPII antigen and pOmpA-5 and pOmpA-7 both encode 451 amino acids of the SERP antigen. In pOmpA-5 and pOmpA-6, the SERP and HRPII partial sequences, respectively, are inserted into the third external loop of OmpA and in pOmpA-3 and pOmpA-7 these antigens are additionally "sandwiched" by the HA hydrophobic moieties in the third external loop domain of OmpA.

After construction of the recombinant vectors, it was shown by DNA sequencing that the malaria specific fragments are in frame with the OmpA and HA coding sequences, respectively. Nucleotide sequencing on double-stranded DNA was performed using the protocol of USB (Cleveland, Ohio) based on the dideoxy-chain-termination method. Sequence data were analyzed and plasmid maps were generated using the UWGCG sequence software as implemented on the MicroVax II (Devereaux et al., Nucl. Acids Res. 12 (1984), 387-395). The following Table I lists these plasmids and details the nature of the OmpA fusion proteins and their molecular weights expected and observed by SDS-PAGE, respectively.

Table I: OmpA plasmids encoding the P. falciparum antigens HRPII und SERP

| plasmid | vector | P. falciparum antigen | construct | MW of fusion protein calculated | found |
|---|---|---|---|---|---|
| pOmpA-3 | pinf4-49 | HRPII | OmpA::HA::HRPII::HA::OmpA | 89 kD | 118 kD |
| pOmpA-5 | phs164-L | SERP | OmpA::SERP::OmpA | 86 kD | 90 kD |
| pOmpA-6 | phs164-L | HRPII | OmpA::HRPII::OmpA | 60 kD | 89 kD |
| pOmpA-7 | pinf4-49 | SERP | OmpA::HA::SERP::HA::OmpA | 117 kD | 110 kD |

## Example 2

The HRPII and SERP OmpA fusion proteins are efficiently expressed and are exposed on the surface of Salmonella typhimurium

S. typhimurium strains phi3730 (LT2-Z, leu, hsdLT, galE, trpD2, rpsL120, delta asdA1, delta [zhf-4::Tn10], metE551, metA22, hsdSA, hsdSB, ilv) and SR-11 (pStSR100 +, gyrA1816, delta cya-1, delta crp-1, delta asdA1, delta [zja-4::Tn10]) carrying plasmid pY248 (S. typhimurium strain phi4072 (pY248)), (Nakajama et al., Biotechnology 6 (1988), 693-697) were transformed with OmpA expression plasmids from E. coli by electroporation using the BioRad Gene Pulser: The restriction-deficient, modification-proficient S. typhimurium strain phi3730 was used to modify pHS164-L and pinf4-49, pOmpA-3, -5, -6 and -7 and after

7

plasmid reisolation, these plasmids were transformed into the restriction-proficient, modification-proficient S. typhimurium strain phi4072 (pY248): 1$\mu$g of DNA was mixed with approximately 2.5 x $10^8$ cells in a precooled cuvette and electroporated at a field strength of approximately 10 kV/cm for 9 msec. The electroporation mix was diluted into 1 ml of SOC medium (0.5 % Bacto yeast extract, 2 % Bacto tryptone, 10 mM NaCl, 2.5 mM KCl, 10 mM $MgCl_2$, 10 mM $MgSO_4$, 20 mM Glucose) and incubated for 1 h at 37 °C in a shaker bath. Aliquots were plated on LB-AMP lacking diaminopimelic acid (DAP) and incubated at 37 °C. Transformants (ASD+/AMP$^r$) contained both plasmids: The ASD marker contained in plasmid pY248 and one of the four OmpA expression plasmids shown in Table 1. Transformation efficiency was 1 x $10^4$/$\mu$g plasmid DNA (control transformations of E. coli strain 490A yielded 1 x $10^8$/$\mu$g plasmid DNA).

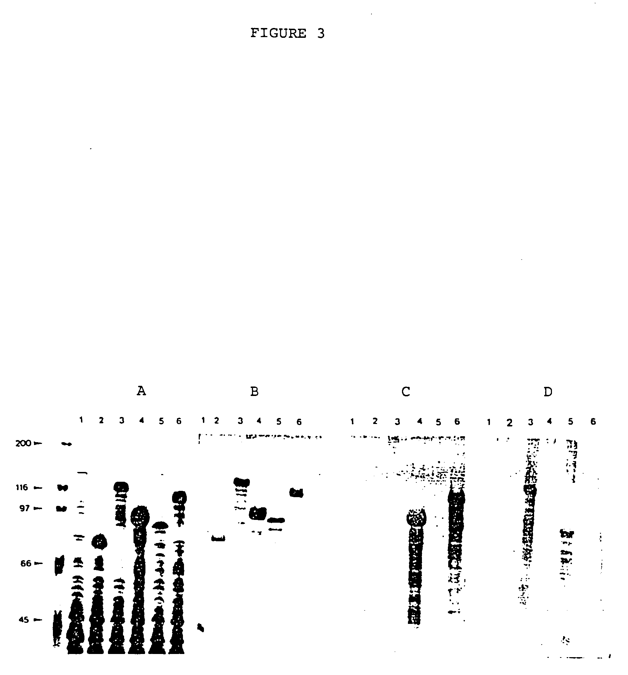

Single transformed colonies were grown in 5 ml LB/AMP medium overnight at 37 °C. 100 $\mu$l of this culture was diluted into 5 ml of the above medium and cells were grown to an $OD_{600}$ of 0.5 - 0.6. Expression was induced by the addition of 1 mM IPTG and the cultures were grown for further three hours. The cells were collected by centrifugation and lysed as described (Amann et al., Gene 69 (1988), 301-305). The final pellets were analyzed by SDS-PAGE. SDS-PAGE analysis of membrane fractions from Salmonella cultures transformed with the expression plasmid is shown in Fig. 3a. It reveals the appearance of new protein bands of the expected molecular weight after IPTG induction. These proteins are only present in the membrane fraction and constitute 10 - 15 % of the total cellular protein.

Western blot analysis of the same samples using an antiserum raised against the 14 C-terminal amino acids (coupled as a haptene to KLH) of OmpA (Fig. 3b) or using monospecific anti-SERP (Fig. 3c) or anti-HRPII (Fig. 3d) rabbit antisera, respectively, prove the identity of the expressed products. In addition to the main band, some degradation is observed.

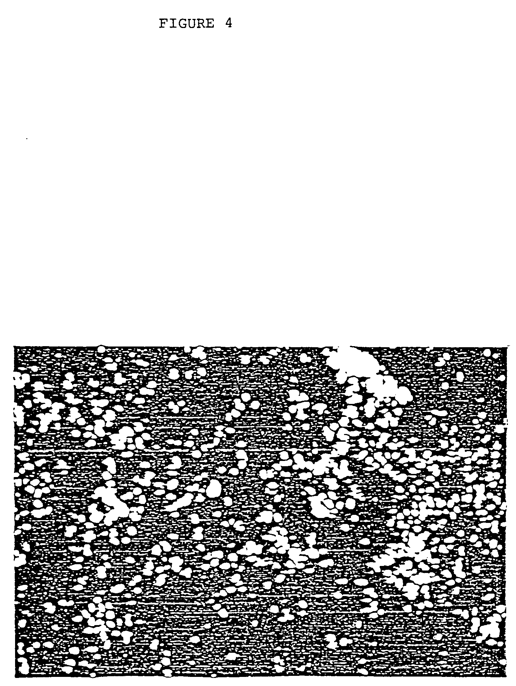

The immunofluorescence technique was used to investigate whether the S. typhimurium cells present the HRPII and SERP antigens on the bacterial surface. To this end, 200 $\mu$l of the induced bacterial culture was centrifuged. Bacteria were washed twice with 300 $\mu$l PBS and suspended in 100 $\mu$l PBS. The anti-HRPII or anti-SERP antiserum was added at a 1 : 50 dilution and the mixture was incubated overnight at 4 °C. Cells were collected by centrifugation, washed as described above and resuspended in 100 $\mu$l PBS. FITC-labeled anti-rabbit IgG from goat was added at a 1 : 50 dilution and the mixture was incubated in the dark for 2 h at room temperature. Cells were collected again, washed as above and resuspended in 50 $\mu$l PBS. 10 $\mu$l of these samples were pipetted onto microscope cover slides and dried at 37 °C. The cover slides were washed carefully with distilled water, dried again and fixed with Moviol. Bacteria were visualized under UV light (300 nm) under a Zeiss microscope and documented by photography. Figure 4 shows the immunofluorescence of cells carrying OmpA-3 probed with an antiserum against HRPII. Recombinant bacteria carrying OmpA-5, -6 and -7 showed similar intensities of immunofluorescence of individual cells and similar numbers of immunofluorescence-positive cells (approx. 90 %) when probed with the corresponding antisera. No fluorescence was obtained when cells carrying the vector plasmids pHS164-L or pinf4-49 or when E. coli cells expressing HRPII or SERP antigens as inclusion bodies within the cell were tested. These studies demonstrate the efficient expression of the malarial antigens on the surface of transformed S. typhimurium bacteria.

**Example 3**

Anti-HRPII and anti-SERP antibody induction in mice upon oral immunization with recombinant Salmonella strains

To investigate the potential of recombinant Salmonella SR-11 strains to induce a humoral immune response against HRPII and SERP antigens expressed on the bacterial surface, about 2 x $10^9$ cells of IPTG induced bacteria carrying the plasmids OmpA-3, -5, -6 or -7 were orally administered at days 0, 7, 14 and 21 to NMRI mice. Mice were starved for five hours before administration. Immediately before feeding bacteria, mice were given orally 0.1 ml sodiumbicarbonate (5 %) in order to neutralize the stomach pH. Blood samples of 100 $\mu$l were drawn retroorbitally at days 0, 14, 28 and 56 and pooled sera were tested for the presence of HRPII and SERP specific IgG and IgM (Fig. 5): ELISA plates coated with purified recombinant E. coli derived SERP- or HRPII-MS2 fusion proteins (5 $\mu$g/ml, Knapp et al., (1988), loc. cit.; Knapp et al., (1989), loc. cit.) were incubated overnight at room temperature and washed. For serum titration, serial dilutions of serum samples were added to the ELISA plates and incubated for 1 h at 37 °C, washed three times and incubated for 1 h at 37 °C with 50 $\mu$l of POD-conjugated anti-mouse antibody. After washing, bound antibodies were visualized with 50 $\mu$l of a o-phenylenediamine/$H_2O_2$ solution. The optical density was measured at 492 nm.

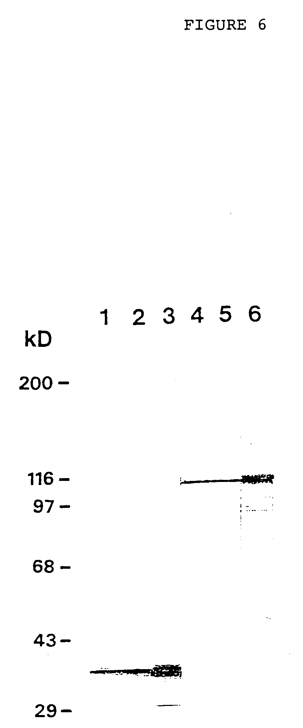

The IgG response against HRPII was faster than against SERP (Fig. 5a). The HA hydrophobic moieties flanking the HRPII and SERP antigens (as present in the respective fusion proteins expressed by pOmpA-3 and pOmpA-7) did not result in significantly higher IgG titers. The anti-HRPII and anti-SERP IgM responses did not show any marked differences and decreased after 14 days (Fig. 5b). Western-blot analyses on P. falciparum polypeptides of the anti-HRPII and anti-SERP mouse sera obtained after oral immunization with recombinant Salmonella strains show the corresponding antigens (Fig. 6). This experiment demonstrates that the oral vaccination of mice with a Salmonella vaccine strain expressing malarial antigens on its surface results in the induction of specific serum IgG antibodies.

**Claims**

1. A recombinant expression vector comprising the following components:
   (a) a strong inducible promoter;
   (b) a hybrid gene encoding a hybrid OmpA protein of gram-negative bacteria;
   (c) a cloning site located in the part of the OmpA gene corresponding to the third external loop of the OmpA protein; and
   (d) a DNA sequence which
      (da) is inserted in frame into the cloning site (c), and
      (db) encodes the malarial antigen HRPII or SERP or a part thereof being capable of inducing a humoral immune response in a mammal, said malarial antigen being preferably derived from Plasmodium falciparum.

2. The recombinant expression vector according to claim 1, which additionally comprises:
   (ca) inserted into said cloning site (c) a DNA sequence encoding amino acids 1 to 48 and 279 to 515 of the Influenza hemagglutinin (HA) protein, containing a first cloning site at a position encoding amino acid 46 of the HA protein and the second cloning site at a position encoding amino acid 400 of the HA protein, wherein said DNA sequence (d) is inserted into at least one of said cloning sites of the DNA sequence (ca).

3. The recombinant expression vector according to claim 1 or 2, wherein said part of HRPII consists of the 189 amino acids contained in the recombinant expression vector pOmpA-6 having the restriction map given in Fig. 1.

4. The recombinant expression vector according to claim 1 or 2, wherein said part of HRPII consists of the 177 amino acids contained in the recombinant expression vector pOmpA-3 having the restriction map given in Fig. 2.

5. The recombinant expression vector according to claim 1 or 2, wherein said part of SERP contains a region being homologous to SH proteases.

6. The recombinant expression vector according to claim 5, which contains amino acids 442 to 892, 573 to 595, or 745 to 787 of SERP.

7. The recombinant expression vector according to claim 1 or 2, wherein said part of SERP contains at least one T-cell epitope.

8. The recombinant expression vector according to claim 7, which contains amino acids 442 to 892 or 640 to 700 of SERP.

9. The recombinant expression vector according to any one of claims 1 to 8, wherein said promoter is the tac-promoter which is under the control of the lac-repressor present upstream on the same expression vector molecule.

10. The recombinant expression vector according to any one of claims 1 to 9, wherein said hybrid gene (b) encodes a hybrid protein wherein
    (ba) the DNA sequence encoding amino acids 1 to 233 is derived from the Shigella dysenteriae OmpA gene; and
    (bb) the DNA sequence encoding amino acids 234 to 325 is derived from the E. coli OmpA gene.

11. The recombinant expression vector according to any one of claims 1 to 10, wherein said cloning site is a polylinker having the nucleotide sequence 5'-ATCGATGCATATGCTGACCCATGGTACCCGGGGAGGCCT-3'.

12. A Salmonella vaccine strain transformed with a recombinant expression vector according to any one of claims 1 to 11.

13. A Salmonella vaccine strain according to claim 12, which is derived from Salmonella typhi or Salmonella typhimurium.

14. A recombinant fusion protein capable of inducing a humoral immune response in mammals, being expressed by a Salmonella vaccine strain according to claim 12 or 13.

15. A vaccine for the prophylaxis of malaria containing a Salmonella vaccine strain according to claim 12 or 13 or a recombinant fusion protein according to claim 14, optionally in combination with a pharmaceutically acceptable carrier and/or diluent.

16. A method for the prophylaxis of malaria comprising administering to a mammal in need thereof a dosage of a Salmonella vaccine strain according to claim 12 or 13 or a recombinant fusion protein according to claim 14, said dosage being suitable for inducing a protective humoral immune response.

FIGURE 1

```
      Lys Ser Met His Met Ser Thr His Gly Thr Arg Gly Gly Leu
   5' gaa tcg atG CAT ATG TCG ACC CAT GGT ACC CGG GGa ggc ctg 3'
        ClaI  NsiI      SalI    NcoI KpnI SmaI     StuI
                 NdeI
```

```
      Ser Arg Val Leu Ile Cys Ile                    Ala Ser Asp Val Asp Pro Gly Gln Asp Leu Glu
B  5' tCT AGA GTA CTC ATA TGC Atc ...... 351bp HA ...... gCT AGC GAC GTC GAC CCG GGC CAA GAT Ctc gag 3'
      XbaI              NsiI                                           SalI  SmaI             XhoI
```

        Linker I                                    Linker II

FIGURE 2

FIGURE 3

FIGURE 4

FIGURE 5

FIGURE 6

FIGURE 7

```
                              EcoRISmaI BamHI HindIIPstI
                              gaattcccgggggatccgtcgacctgcagga
                                                    ------------a
         .            .            .            .            .            .
CATGTAGCCGATGCCCATCATGCTCATCATGCAGCCGATGCCCATCATGCTCATCATGCA
H  V  A  D  A  H  H  H  A  H  H  A  A  D  A  H  H  H  A  H  H  A
-------------------
         .            .            .            .            .            .
GCCGATGCCCATCATGCTCATCATGCAGCCTATGCCCATCATGCTCATCATGCAGCCGAT
A  D  A  H  H  H  A  H  H  A  A  Y  A  H  H  A  H  H  A  A  D
         .            .            .            .            .            .
GCCCATCATGCTCATCATGCTCACCATGCAGCCGATGCCCATCACGCTCATCATGCAGCC
A  H  H  A  H  H  A  H  H  A  A  D  A  H  H  A  H  H  A  A
         .            .            .            .            .            .
GATGCCCATCATGCTCACCATGCAGCTGATGCTCATCACGCTCATCATGCAGCCGATGCC
D  A  H  H  A  H  H  A  A  D  A  H  H  A  H  H  A  A  D  A
         .            .            .            .            .            .
CATCATGCTCATCATGCAGCCGATGCCCATCATGCTCACCATGCATCCGATGCTCATCAT
H  H  A  H  H  A  A  D  A  H  H  A  H  H  A  S  D  A  H  H
         .            .            .            .            .            .
GCAGCTGATGCTCACCATGCAGCCTATGCCCATCATGCTCATCATGCTCATCATGCATCC
A  A  D  A  H  H  A  A  Y  A  H  H  A  H  H  A  H  H  A  S
         .            .            .            .            .            .
GATGCTCATCATGCAGCTGATGCTCACCATGCAGCTTATGCCCATCACGCTCATCATGCA
D  A  H  H  A  A  D  A  H  H  A  A  Y  A  H  H  A  H  H  A
         .            .            .            .            .            .
GCTGATGCTCATCATGCAGCCGATGCCCATCATGCTCACCATGCAACCGATGCTCATCAC
A  D  A  H  H  A  A  D  A  H  H  A  H  H  A  T  D  A  H  H
         .            .            .            .            .  *Bal31  .
GCTCACCATGCAGCCGATGCTCACCATGCAACCGATGCTCACCATGCAGCCGCACACCAC
A  H  H  A  A  D  A  H  H  A  T  D  A  H  H  A  A  A  H  H
         .            .            .            .    PstI      HindIII
GAAGCCGCCACACATTGCCTACGCCATTAAATTTATTTAATAActctgcagccaagctt
E  A  A  T  H  C  L  R  H
-------------------------------------       --------------------
                                     ggtacc
                                     KpnI
```

FIGURE 8

```
   1 TCTAGATTTATTCTCTTATGAGAATGTACAAAAAAAAAAAAAAAAAATTAAAATAAAAAAATAAAAATTAAATAATTTAAATAATATAATATATTTATATTAAATTTATATATATATAT

 121 ATAATAAATTTTTTAATTTAAAATTAAATTAGATTGTCCAAAAAAAAAAATAAAAAAATAAATATATATATATTATAAAATACATATATTATAACATAAAGAAAAATTAAATAAATCA

 241 AACATATTCAAAAAAATTAAAGTTCTTAAAATATTATATAACTTAATATTCATATATCAAAATGAAGTCATATATTTCCTTGTTTTTCATATTGTGTAAGAATGAAAAAAAAAAAAAAA
                                                                      MetLysSerTyrIleSerLeuPhePheIleLeuL→      11

 361 GGAAAAGGAAAAGGAAAAGAAAAGGAAAAGAAAAAGAAAAACAAATATGTAAAAATATAATTATTATAATAATAATAATATTTTTACGCATACACAAACATTTGTCATTATTTT

 481 TTTTTAGGTGTTATATTTAACAAAAATGTTATAAAATGTACAGGAGAAAGTCAAACAGGTAATACAGGAGGAGGTCAAGCAGGTAATACAGGAGGAGGTCAAGCAGGTAATACAGTAGGA
       →LysValIlePheAsnLysAsnValIleLysCysThrGlyGluSerGlnThrGlyAsnThrGlyGlyGlyGlnAlaGlyAsnThrGlyGlyGlyGlnAlaGlyAsnThrValGly   49

 601 GATCAAGCAGGTAGTACAGGAGGAAGTCCACAAGGTAGTACGGGAGCAAGTCAACCCGGAAGTTCCGAACCAAGCAATCCTGTAAGTTCCGGACATTCTGTAAGTACTGTATCAGTATCA
       AspGlnAlaGlySerThrGlyGlySerProGlnGlySerThrGlyAlaSerGlnProGlySerSerGluProSerAsnProValSerSerGlyHisSerValSerThrValSerValSer   89

 721 CAAACTTCAACTTCTTCAGAAAAACAGGATACAATTCAAGTAAAATCAGCTTTATTAAAAGATTATATGGGTTTAAAGTTACTGGTCCATGTAACGAAAATTTCATAATGTTCTTAGTT
       GlnThrSerThrSerSerGluLysGlnAspThrIleGlnValLysSerAlaLeuLeuLysAspTyrMetGlyLeuLysValThrGlyProCysAsnGluAsnPheIleMetPheLeuVal   129

 841 CCTCATATATATATTGATGTTGATACAGAAGATACTAATATCGAATTAAGAACAACATTGAAAGAAACAAATAATGCAATATCATTTGAATCAAACAGTGGTTCATTAGAAAAAAAAAAA
       ProHisIleTyrIleAspValAspThrGluAspThrAsnIleGluLeuArgThrThrLeuLysGluThrAsnAsnAlaIleSerPheGluSerAsnSerGlySerLeuGluLysLysLys   169

 961 TATGTAAAACTACCATCAAATGGTACAACTGGTGAACAAAGTTCTAGTTCAAGTTCAAGTTCTAGTTCAAATTCTAGTTCAAGTTCAAGTTCAAGTTCAAGTTCTAGTTCAAGTTCAAGT
       TyrValLysLeuProSerAsnGlyThrThrGlyGluGlnSerSerSerSerSerSerSerSerSerSerAsnSerSerSerSerSerSerSerSerSerSerSerSerSerSerSerSer   209

1081 TCAAGTTCTAGTTCAAGTTCTAGTTCAAGTTCAGAAAGTCTTCCTGCTAATGGACCTGATTCCCCTACTGTTAAACCGCCAAGAAATTTACAAAATATATGTGAAACTGGAAAAAACTTC
       SerSerSerSerSerSerSerSerSerSerGluSerLeuProAlaAsnGlyProAspSerProThrValLysProProArgAsnLeuGlnAsnIleCysGluThrGlyLysAsnPhe   249

1201 AAGTTGGTAGTATATATTAAGGAGAATACATTAATAATTAAATGGAAAGTATACGGAGAAACAAAAGATACTACTGAAAGTATAAAAAAATAACCGAATAAAACAATAATAATAATACTTT
       LysLeuValValTyrIleLysGluAsnThrLeuIleIleLysTrpLysValTyrGlyGluThrLysAspThrThrGluA→     275

1321 TTTCTTTTTTTGATTGATTATTTTTATATTTTCATAAGAAAATGTCATTATACATACAACTACTATCAATTATGTATATTTTGTTATTATTTTATATTATTATTATTATTTTATTTTATTTATT

1441 TATTTTTTTTTTAGATAACAAAGTTGATGTAAGAAAGTATTTGATAAATGAAAAGGAAACCCCATTTACTAGTATACTAATACATGCGTATAAAGAACATAATGGAACAAACTTAATAGA
       →SnAsnLysValAspValArgLysTyrLeuIleAsnGluLysGluThrProPheThrSerIleLeuIleHisAlaTyrLysGluHisGlyThrAsnLeuIleGl   311

1561 AAGTAAAAACTACGCATTAGGATCAGACATTCCAGGTAAATAACAATAAAGAGGAATATACAAATGTATGAATATATATGCATAATCAAAATGTAATATATATATATATATATATATATA
       uSerLysAsnTyrAlaLeuGlySerAspIleProd→

1681 TATATACATATATTATATATTTTTTTAATTTTTTTTGTTTTAGAAAAATGTGATACCTTAGCTTCCAATTGCTTTTTAAGTGGTAATTTTAACATTGAAAAATGCTTTCAATGTGCTCTTTTA
       →luLysCysAspThrLeuAlaSerAsnCysPheLeuSerGlyAsnPheAsnIleGluLysCysPheGlnCysAlaLeuLeu   349

1801 GTAGAAAAAGAAAATAAAAATGACGTATGTTACAAATACCTATCTGAAGATATTGTAAGTAAATTCAAAGAAATAAAAGCTGAGACAGAAGATGATGATGAAGATGATTATACTGAATAT
       ValGluLysGluAsnLysAsnAspValCysTyrLysTyrLeuSerGluAspIleValSerLysPheLysGluIleLysAlaGluThrGluAspAspAspGluAspAspTyrThrGluTyr   389

1921 AAAATTAACAGAATCTATTGATAATATATTAGTAAAAATGTTTAAAACAAATGAAAATAATGATAAATCAGAATTAATAAAATTAGAAGAAGTAGATGATAGTTTGAAATTAGAATTAATG
       LysLeuThrGluSerIleAspAsnIleLeuValLysMetPheLysThrAsnGluAsnAsnAspLysSerGluLeuIleLysLeuGluGluValAspAspSerLeuLysLeuGluLeuMet   429

2041 AATTACTGTAGTTTACTTAAAGACGTAGATACAACAGGTACCTTAGATAATTATGGGATGGGAAATGAAATGGATATATTTAATAACTTAAAGAGATTATTAATTTATCATTCAGAAGAA
       AsnTyrCysSerLeuLeuLysAspValAspThrThrGlyThrLeuAspAsnTyrGlyMetGlyAsnGluMetAspIlePheAsnAsnLeuLysArgLeuLeuIleTyrHisSerGluGlu   469

2161 AATATTAATACTTTAAAAAAATAAATTCCGTAATGCAGCTGTATGTCTTAAAAAATGTTGATGATTGGATTGTAAATAAGAGAGGTTTAGTATTACCTGAATTAAATTATGATTTAGAATAT
       AsnIleAsnThrLeuLysAsnLysPheArgAsnAlaAlaValCysLeuLysAsnValAspAspTrpIleValAsnLysArgGlyLeuValLeuProGluLeuAsnTyrAspLeuGluTyr   509

2281 TTCAATGAACATTTATATAATGATAAAAATTCTCCAGAAGATAAAGATAATAAAGGAAAAGGTGTCGTACATGTTGATACAACTTTAGAAAAAGAAGATACTTTATCATATGATAACTCA
       PheAsnGluHisLeuTyrAsnAspLysAsnSerProGluAspLysAspLysLysGlyLysGlyValHisValAspThrThrLeuGluLysGluAspThrLeuSerTyrAspAsnSer   549

2401 GATAATATGTTTTGTAATAAAGAATATTGTAACAGATTAAAAGATGAAAATAATTGTATATCTAATCTTCAAGTTGAAGATCAAGGTAATTGTGATACTTCATGGATTTTTGCTTCAAAA
       AspAsnMetPheCysAsnLysGluTyrCysAsnArgLeuLysAspGluAsnAsnCysIleSerAsnLeuGlnValGluAspGlnGlyAsnCysAspThrSerTrpIlePheAlaSerLys   589

2521 TATCATTTAGAAACTATTAGATGTATGAAAGGATATGAACCTACCAAAATTTCTGCTCTTTATGTAGCTAATTGTTATAAAGGTGAACATAAAGATAGATGTGATGAAGGTTCTAGTCCA
       TyrHisLeuGluThrIleArgCysMetLysGlyTyrGluProThrLysIleSerAlaLeuTyrValAlaAsnCysTyrLysGlyGluHisLysAspArgCysAspGluGlySerSerPro   629

2641 ATGGAATTCTTACAAATTATTGAAGATTATGGATTCTTACCAGCAGAATCAAATTATCCATATAACTATGTGAAAGTTGGAGAACAATGTCCAAAGGTAGAAGATCACTGGATGAATCTA
       MetGluPheLeuGlnIleIleGluAspTyrGlyPheLeuProAlaGluSerAsnTyrProTyrAsnTyrValLysValGlyGluGlnCysProLysValGluAspHisTrpMetAsnLeu   669

2761 TGGGATAATGGAAAAAATCTTACATAACAAAAATGAACCTAATAGTTTAGATGGTAAGGGATATACTGCATATGAAAGTGAAAGATTTCATGATAATATGGATGCATTTGTTAAAATTATT
       TrpAspAsnGlyLysIleLeuHisAsnLysAsnGluProAsnSerLeuAspGlyLysGlyTyrThrAlaTyrGluSerGluArgPheHisAspAsnMetAspAlaPheValLysIleIle   709

2881 AAAACTGAAGTAATGAATAAAGGTTCAGTTATTGCATATATTAAAGCTGAAAATGTTATGGGATATGAATTTAGTGGAAAGAAAGTACAGAACTTATGTGGTGATGATCAGCTGATCAT
       LysThrGluValMetAsnLysGlySerValIleAlaTyrIleLysAlaGluAsnValMetGlyTyrGluPheSerGlyLysLysValGlnAsnLeuCysGlyAspAspThrAlaAspHis   749

3001 GCAGTTAATATTGTTGGTTATGGTAATTATGTGAATAGCGAAGGAGAAAAAAATCCTATTGGATTGTAAGAAACAGTTGGGGTCCATATTGGGGAGATGAAGGTTATTTTAAAGTAGAT
       AlaValAsnIleValGlyTyrGlyAsnTyrValAsnSerGluGlyGluLysLysSerTyrTrpIleValArgAsnSerTrpGlyProTyrTrpGlyAspGluGlyTyrPheLysValAsp   789

3121 ATGTATGGACCAACTCATTGTCATTTTAACTTTATTCACAGTGTTGTTATATTCAATGTTGATTTACCTATGAATAATAAAACAACTAAAAAAGAATCAAAAATATATGATTATTATTTA
       MetTyrGlyProThrHisCysHisPheAsnPheIleHisSerValValIlePheAsnValAspLeuProMetAsnAsnLysThrThrLysLysGluSerLysIleTyrAspTyrTyrLeu   829

3241 AAGGCCTCTCCAGAATTTTATCATAACCTTTACTTTAAGAATTTTAATGTTGGTAAGAAAAATTTATTCTCTGAAAAGGAAGATAATGAAAACAACAAAAAATTAGGTAACAACTATATT
       LysAlaSerProGluPheTyrHisAsnLeuTyrPheLysAsnPheAsnValGlyLysLysAsnLeuPheSerGluLysGluAspAsnGluAsnAsnLysLysLeuGlyAsnAsnTyrIle   869

3361 ATATTCGGTCAAGATACGGCAGGATCAGGACAAAGTGGAAAGGAAAGCAATACTGCAGGAACTTCAAATGAAGTCTCAGAACGTGTTCATGTTTATCACATATTAAAA
       IlePheGlyGlnAspThrAlaGlySerGlyGlnSerGlyLysGluSerAsnThrAlaLeuGluSerAlaGlyThrSerAsnGluValSerGluArgValHisValTyrHisIleLeuLys   909

3481 CATATAAAGGATGGCAAAAATAAGAATGGGTATGCGTAAATATATAGATACACAAGATGTAAATAAGAAACATTCTTGTACAAGATCCTATGCATTTAATCCAGAGAATTATGAAAAATGT
       HisIleLysAspGlyLysIleArgMetGlyMetArgLysTyrIleAspThrGlnAspValAsnLysLysHisSerCysThrArgSerTyrAlaPheAsnProGluAsnTyrGluLysCys   949

3601 GTAAATTTATGTAATGTGAACTGGAAAACATGCGAGGAAAAAACATCACCAGGACTTTGTTTATCCAAATTGGATACAAATAACGAATGTTATTTCTGTTATGTATAAAATAATATAACA
       ValAsnLeuCysAsnValAsnTrpLysThrCysGluGluLysThrSerProGlyLeuCysLeuSerLysLeuAspThrAsnAsnGluCysTyrPheCysTyrVal***   984

3721 AAAAAAAAAAAAAAAAAATATTTTTTTTTTATGTATCCTTTAATTTTTAAATAGGGCATAACTCTCCATTATTCATTTTATTAAGGTAGTATAATATCTTTAATTTATCATGTACCTCTAT

3841 AAATATATATAAAATTATATTATTATTATTTTTTTTTTTTAAGAATTATTTTTATTCATGTAAATATAATTCTTTTTTTTTTTTTTTTTTTTTTTTTTTTAAAAAAAAATACACGATAGTTGT

3961 ACATTAAATGTATAC
```

European Patent Office

**PARTIAL EUROPEAN SEARCH REPORT**
which under Rule 45 of the European Patent Convention
shall be considered, for the purposes of subsequent
proceedings, as the European search report

Application number

EP 90 11 7344

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,Y | EP-A-0 355 737 (BEHRINGWERKE)<br><br>* The whole document *<br><br>-- | 1-15 | C 12 N 15/62<br>C 12 N 15/30<br>C 12 N 15/44<br>C 12 N 15/31<br>C 12 N 1/21//<br>A 61 K 39/112<br>A 61 K 39/015<br>A 61 K 39/145 |
| D,Y | EP-A-0 315 085 (BEHRINGWERKE)<br><br>* The whole document *<br><br>-- | 1-4,<br>9-15 | |
| D,Y | EP-A-0 283 882 (BEHRINGWERKE)<br><br>* The whole document *<br><br>-- | 5-8 | |
| | ./. | | |

**TECHNICAL FIELDS SEARCHED (Int. Cl.4)**

C 12 N
C 07 K
A 61 K

## INCOMPLETE SEARCH

The Search Division considers that the present European patent application does not comply with
the provisions of the European Patent Convention to such an extent that it is not possible to carry
out a meaningful search into the state of the art on the basis of some of the claims.

Claims searched completely:     1-15
Claims searched incompletely:
Claims not searched:              16
Reason for the limitation of the search:

Method for treatment of the human or animal
body by surgery or therapy (see Art. 52(4)
of the European Patent Convention).

| Place of search | Date of completion of the search | Examiner |
|---|---|---|
| THE HAGUE | 06-05-1991 | SKELLY |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO Form 1505.1. 03.82

| DOCUMENTS CONSIDERED TO BE RELEVANT | | | CLASSIFICATION OF THE APPLICATION (Int. Cl.4) |
|---|---|---|---|
| Category | Citation of document with indication, where appropriate, of relevant passages | Relevant to claim | |
| D,A | NATURE, vol. 340, no. 6235, 24th August 1989, page 604, London, GB; D.G. HIGGENS et al.: "Malarial proteinase?" <br><br> * The whole article * | 5-8 | |
| D,A | MOLECULAR AND BIOCHEMICAL PARASI-TOLOGY, vol. 32, 1989, pages 73-84, Elsevier Science Publishers; B. KNAPP et al.: "Molecular cloning, genomic structure and localization in a blood stage antigen of plasmodium falciparum characterized by a serine stretch" <br><br> -- | | TECHNICAL FIELDS SEARCHED (Int Cl 4) |
| A | WO-A-87 06 590 (BIOENTERPRISES PTY) <br><br> * Claims 19-43 * | 1-15 | |